Europäisches Patentamt

European Patent Office

Office européen des brevets

(11) Publication number: **0 146 925**
**B1**

(12) EUROPEAN PATENT SPECIFICATION

(45) Date of publication of patent specification: **04.11.87**

(21) Application number: **84115870.2**

(22) Date of filing: **19.12.84**

(51) Int. Cl.⁴: **C 07 C 19/045, C 07 C 17/156**

(54) Method for the oxychlorination of ethylene.

(30) Priority: **22.12.83 DE 3346464**

(43) Date of publication of application:
**03.07.85 Bulletin 85/27**

(45) Publication of the grant of the patent:
**04.11.87 Bulletin 87/45**

(84) Designated Contracting States:
**FR GB IT SE**

(56) References cited:
**DE-A-1 493 213**
**FR-A-2 388 784**

(73) Proprietor: **WACKER-CHEMIE GMBH**
**Prinzregentenstrasse 22**
**D-8000 München 22 (DE)**

(72) Inventor: **Schmidhammer, Ludwig, Dr.**
**Pappelweg 5**
**D-8261 Haiming (DE)**
Inventor: **Strasser, Rudolf, Dr.**
**Lindacherstrasse 58**
**D-8263 Burghausen (DE)**
Inventor: **Hirschmann, Peter**
**Jägerweg 5**
**D-8263 Burghausen (DE)**
Inventor: **Haselwarter, Klaus**
**Marktlerstrasse 7**
**D-8263 Burghausen (DE)**
Inventor: **Dummer, Gerhard**
**Lohnerstrasse 12**
**D-8269 Burgkirchen (DE)**

(74) Representative: **Kraus, Walter, Dr. et al**
**Patentanwälte Kraus, Weisert & Partner**
**Thomas-Wimmer-Ring 15**
**D-8000 München 22 (DE)**

Courier Press, Leamington Spa, England.

## Description

### Background and prior art

The invention concerns a method for the oxychlorination of ethylene in a fixed bed system which consists of at least two serially connected reactors, in the presence of a supported cupric chloride catalyst.

Oxychlorination reactions in the presence of Deacon type catalysts are appropriately carried out in a temperature range of approximately 200—350°C. While a temperature of approximately 200°C should be maintained in order to obtain adequate conversion rates, temperatures of approximately 350°C should not be exceeded since catalyst damage, selectivity losses or even explosive operating conditions can occur. Because of the strongly exothermic character of the oxychlorination reaction and the associated tendency for the formation of highly overheated zones (hot spots) in the catalyst bed, considerable problems arise in operating within the cited upper limit of the temperature range.

Therefore, a method has been proposed in Federal Republic of Germany Offenlegungsschrift 14 93 213 in which the oxychlorination is carried out in a system of several serially connected reactors and the total quantity of oxygen required for oxychlorination is divided up among the corresponding number reactors. Dilution of the Deacon catalyst by the addition of an inert material to avoid overheating in the catalyst bed was also proposed.

In accordance with U.S. Patent 3,892,816, oxychlorination is carried out in a multireactor system in the presence of a large ethylene excess. Pure oxygen must be used in this operation; its addition, together with addition of hydrogen chloride, is distributed over the corresponding number of reactors. Excess, unreacted ethylene is recycled. However, such a recycling method is extremely costly and subject to disturbance. Also, the cost efficiency of the method can be questioned since the total oxygen requirement must be used in the form of relatively expensive pure oxygen.

The methods in accordance with the prior art have in common the control of the strongly exothermic oxychlorination reaction by dilution—either by diluting the reaction mixture with large quantities of inert gases (for example, with nitrogen from the air) or by using a large excess of one of the reaction components (for example, ethylene) or by adding inert material to the catalyst. Reaction control is therefore carried out at the expense of the space-time performance of a given installation. This represents a conflict of goals, that is, the attainment of as high a production rate as possible in a given installation without having to accept disadvantages such as reductions in selectivity, etc. It is solved by the known methods in a manner which is not very economical.

### Summary of the invention

According to this invention, ethylene is converted to 1,2-dichloroethane by oxychlorination with hydrogen chloride and an oxygen-containing gas in a multiple reactor fixed bed catalyst bed system containing a cupric chloride catalyst, in which the total amounts of ethylene and oxygen, introduced into the process, are respectively from 110% to 120% and from 105% to 115% of stoichiometric, based on hydrogen chloride, and the oxygen-containing gas is air which has been enriched to contain from 28 to 60 volume % oxygen.

### Detailed description of the invention

Surprisingly, it has now been found that in processes of this type it is possible to keep down the combustion rate with an increased oxygen fraction based on the total quantity of reactants. Despite the method-related reduction in contact times, the catalyst-specific hydrogen chloride conversion is increased by use of the method according to the invention. Furthermore, it was unexpected that, despite a low inert gas fraction and an increased production rate, no impermissibly high hot-spot temperatures appear in the catalyst bed.

The oxygen content of the oxygen-enriched air is 28—60, preferably 30 to 35 volume %.

The oxychlorination is carried out in a fixed bed reactor system consisting of at least two serially connected reactors. Two- or three- and particularly three-reactor installations are preferred. Multitubular reactors of approximately the same size are used with an appropriate vertical arrangement for flow from top to bottom. The tubular reactors are equipped with cooling jackets. Water under increased pressure is used in particular as the cooling medium.

The tubes contain a supported catalyst with cupric chloride as the active component and alumina, silica gel, aluminosilicate, etc. as supports. The support material may be present in the form of spheres, cones, cubes, hollow strands, etc. The catalyst preferably has an activity profile within each reactor which increases in the direction of flow and which may be prepared by increasing the quantity of active substance, based on the total weight of catalyst, in the direction of flow. For a given active substance concentration at the reactor entrance of 5 to 7 wt.%, the active substance concentration increases continuously or discontinuously up to the reactor exit by up to a factor of 2.5 to 4.

In addition to the cupric chloride active substance, the catalyst can also contain promoters such as the chlorides of potassium, magnesium, calcium and silver. The concentration of promoters is an essentially uniform 1.5 to 3.5 wt.% based on the total weight of the catalyst.

The ethylene, oxygen and hydrogen chloride reaction components are used in quantities such that, based on the stoichiometry of the oxychlorination reaction, there is a total ethylene excess of 10 to 20 mole

2

**0 146 925**

% and an oxygen excess of 5 to 15 mole %, based in each case on the total quantity of hydrogen chloride used.

Approximately equal parts of the oxygen-enriched air (28 to 60 vol. % oxygen) are conducted to the individual reactors. On the other hand, the total quantity of ethylene is added in the first of the serially connected reactors. In two-reactor installations, the total quantity of hydrogen chloride is also added in the first reaction; the hydrogen chloride is divided into approximately equal parts among the first two serially connected reactors for installations with three or more reactors.

The reaction components in two- and three-reactor systems are preferably supplied according to the following scheme. The percentage refers in each case to the total quantities of the individual reaction components.

### Two-reactor system

|  | Reactor 1 | Reactor 2 |
|---|---|---|
| Ethylene | total volume | — |
| Hydrogen chloride | total volume | — |
| Oxygen | 40—45% of total volume | 55—60% of total volume |

### Three-reactor system

|  | Reactor 1 | Reactor 2 | Reactor 3 |
|---|---|---|---|
| Ethylene | total volume | — | — |
| Hydrogen chloride | 70—80% of total | 20—30% | — |
| Oxygen | 35—45% of total | 38—40% | 20—27% |

Small quantities of steam can also be added to the reaction mixtures before the first reactor. This supply takes place, for example, together with the oxygen-enriched air in quantities of 0.05 to 0.20 mole % steam based on the total quantity of hydrogen chloride.

The reaction components may be supplied individually or in a mixture according to the supply scheme described above after pre-heating to temperatures of 100 to 200°C.

The reaction temperatures in the reactor lie at 200 to 350°C and preferably at 200 to 300°C. The system pressure in the reactors is 3 to 8 bar abs.

The reaction mixture exiting the reaction system is worked up in a known manner by cooling, condensing and separation into organic and inorganic phases.

According to the method of the invention, it is possible without high investment costs to increase the production capacity of an existing installation up to 50% in comparison to the installation operated with air from the ambient atmosphere. For customary commercially used multitubular reactors (with inside tube diameters of 25—30 mm), an HCl throughput of 2.2—2.4 $Nm^3$ per tube per hour is attained in conventional methods; however, an HCl throughput of 3.5—3.7 $Nm^3$ per tube per hour is attained according to the method of the invention. Other advantages occur during the work-up of the reaction mixture leaving the reactor installations, particularly in the condensation stage, because of the small inert fraction.

### Example

A reaction installation was used which consisted of three serially connected multitubular reactors of the same size. Each reactor contained 1570 nickel tubes with an inside diameter of 27.5 mm. The nickel tubes contained catalyst with an activity profile which increased in the direction of flow. Aluminum oxide was used as the support material; it was present in the form of 3—5 mm pellets. An activity profile increasing in the direction of flow was realized by a concentration of cupric chloride of 6.5 wt.% in the entrance third of each reactor; 11 wt.% in the middle third and 19 wt.% in the final third. In addition to the cupric chloride active component, the catalyst also uniformly contained 2 wt.% potassium chloride. The catalyst volume was 10.2 $m^3$.

The nickel tubes were surrounded with an external jacket in which water was used as the cooling medium to remove the released reaction heat. The water was under a pressure of 19 bar abs. in the first reactor, 21 bar abs. in the second reactor and 24 bar abs. in the third reactor.

Air which had been enriched to an oxygen content of 30 vol. % by the addition of pure oxygen was used as the oxygen source.

A mixture of 175 kmol/h hydrogen chloride, 144 kmol/h ethylene and 84 kmol/h air enriched to 30 vol. % oxygen was fed to the first reactor at regulated pressures of 8 bar abs. The oxygen-enriched air was

3

0 146 925

injected with 28 kmol/h steam and this mixture was pre-heated to 180°C before mixing with the other reaction components. The temperature of the reaction mixture before entering the reactor was 130°C.

Before entering the second reactor, the reaction mixture exiting the first reactor was mixed with a mixture of 75 kmol/h hydrogen chloride and 90 kmol/h air enriched to 30 vol. % oxygen (pre-heated to a temperature of 180°C). Finally, another 51 kmol/h air enriched to 30 vol. % oxygen was supplied to the reaction mixture between the second and third reactors.

The system pressure before the first reactor was 6.5 bar abs. the pressure drop across the reactors was 0.28 bar in the first reactor, 0.38 bar in the second reactor and 0.41 bar in the third reactor. The hot-spot temperatures were 282°C in the first reactor, 275°C in the second reactor and 260°C in the third reactor.

The reaction mixture exited the third reactor at a temperature of 220°C and was cooled and condensed. The liquid condensate was separated into organic and aqueous phases in a downstream decanter.

The organic phase had the following composition:

| | |
|---|---|
| ethyl chloride | 0.29 wt.% |
| 1,1-dichloroethane | 0.01 wt.% |
| chloroform | 0.04 wt.% |
| carbon tetrachloride | 0.09 wt.% |
| dichloroethylene | 0.01 wt.% |
| 1,1,1-trichloroethane | 0.40 wt.% |
| chloral | 0.26 wt.% |
| 1,2-dichloroethane | 98.75 wt.% |
| other | 0.10 wt.% |

An analysis of the condensed aqueous phase produced the following:

| | |
|---|---|
| hydrogen chloride | 1.94 wt.% |
| chloral hydrate | 0.45 wt.% |
| 1,2-dichloroethane | 0.83 wt.% |

A hydrogen chloride conversion of 99.4% is thus calculated from the hydrogen chloride content.

The analysis of the uncondensable waste gas flow produced the following (without entrained steam, ethyl chloride and 1,2-dichloroethane):

| | |
|---|---|
| nitrogen | 87.10 wt.% |
| oxygen | 2.10 wt.% |
| ethylene | 8.07 wt.% |
| carbon monoxide | 1.53 vol.% |
| carbon dioxide | 1.30 vol.% |

The waste gas quantity was 4050 $Nm^3$/h.

A specific combustion ratio of 9.4 $Nm^3$ $CO+CO_2$/1000 kg of produced raw 1,2-dichloroethane resulted from the carbon monoxide and carbon dioxide content.

The production rate is 12,200 kg 1,2-dichloroethane per hour. The space-time performance was 1196 kg 1,2-dichloroethane per $m^3$ catalyst×hour based on all three reactors.

Comparison Example

An operation according to the Example was repeated with the modification that, instead of air enriched to 30 vol. % oxygen, air from the ambient atmosphere was used (corresponding to an oxygen content of 20.9 vol. %).

It was possible to supply the reactor installation with only 98 kmol/h ethylene, 179 kmol/h hydrogen chloride and 88 kmol/h air together with 19 kmol/h steam to the first reactor; 51 kmol/h hydrogen chloride and 98 kmol/h air to the second reactor and 45 kmol/l air to the third reactor. Because of the large pressure losses in the reactors, a system pressure of 6.8 bar abs. had to be established in the first reactor. A further throughput increase was not possible because of the small pressure difference with respect to the entry flows.

The hydrogen chloride conversion was 98.9%.

The specific combustion rate was 9.3 $Nm^3$ $CO+CO_2$/1000 kg of produced raw dichloroethane. The production rate was 8260 kg 1,2-dichloroethane per hour. The space-time performance was 810 kg 1,2-dichloroethane per $m^3$ catalyst×hour based on all three reactors.

The waste gas quantity was 4310 $Nm^3$/h, corresponding to a specific waste gas quantity of 521.8 $Nm^3$ per 1000 kg of produced dichloroethane as compared with only 332 $Nm^3$ in the operation according to the invention.

4

**Claims**

1. A process for the production of 1,2-dichloroethane by oxychlorination of ethylene with hydrogen chloride and an oxygen-containing gas in a multiple-reactor fixed bed catalyst system in which the total amounts of ethylene and oxygen introduced into the process are respectively from 110% to 120% and from 105% to 115% of stoichiometric, with respect to hydrogen chloride and the oxygen-containing gas is air which has been enriched to contain from 28 to 60 volume % oxygen.

2. A process according to Claim 1 in which the catalyst comprises cupric chloride.

3. A process according to Claim 1 in which the reactors are multitubular reactors in which the tubes are packed with the catalyst.

4. A process according to Claim 3 in which the strength of the catalyst in each reactor increases from the reactor inlet to the outlet.

5. A process according to Claim 1 in which the air is enriched to contain from 30 to 35 volume % oxygen.

**Patentansprüche**

1. Verfahren zur Herstellung von 1,2-Dichlorethan durch Oxychlorierung von Ethylen mit Chlorwasserstoff und einem Sauerstoff enthaltenden Gas in einem Katalysatorsystem mit mehreren Reaktoren und stationärer Schicht, dadurch gekennzeichnet, daß die Gesamtmengen von Ethylen und Sauerstoff, die in das Verfahren eingeleitet werden, von 110 bis 120% bzw. 105 bis 115% der stöchiometrischen Menge, bezogen auf Chlorwasserstoff, betragen und daß das Sauerstoff enthaltende Gas Luft ist, welche so angereichert worden ist, daß sie von 28 bis 60 Vol.-% Sauerstoff enthält.

2. Verfahren nach Anspruch 1, dadurch gekennzeichnet, daß der Katalysator Kupfer(II)chlorid enthält.

3. Verfahren nach Anspruch 1, dadurch gekennzeichnet, daß die Reaktoren Reaktoren mit mehreren Röhren sind, wobei die Röhren mit dem Katalysator gefüllt sind.

4. Verfahren nach Anspruch 3, dadurch gekennzeichnet, daß sich die Stärke des Katalysators in jedem Reaktor vom Reaktoreinlaß zum Auslaß erhöht.

5. Verfahren nach Anspruch 1, dadurch gekennzeichnet, daß die angereicherte Luft von 30 bis 35 Vol.-% Sauerstoff enthält.

**Revendications**

1. Un procédé de préparation de 1,2-dichloroéthane par oxychloruration d'éthylène à l'aid d'acide chlorhydrique et d'un gaz contenant de l'oxygène dans un système de catalyseur en lit fixe à réacteurs multiples, dans lequel les quantités totales d'éthylène et d'oxygène introduites dans le procédé sont respectivement de 110 à 120%, et de 105 à 115% des quantités stoechiométriques par rapport à l'acide chlorhydrique, et le gaz contenant de l'oxygène et de l'air que l'on a enrichi pour qu'il contienne de 28 à 60% en volume d'oxygène.

2. Un procédé selon la revendication 1, dans lequel le catalyseur comprend du chlorure cuivrique.

3. Un procédé selon la revendication 1, dans lequel les réacteurs sont des réacteurs multi-tubulaires dans lequel les tubes sont garnis de catalyseur.

4. Un procédé selon la revendication 3, dans lequel la force du catalyseur dans chaque réacteur augmente de l'entrée à la sortie du réacteur.

5. Un procédé selon la revendication 1, dans lequel l'air est enrichi de façon à contenir de 30 à 35% en volume d'oxygène.